# EUROPÄISCHE PATENTSCHRIFT

(11) **EP 1 395 586 B1**
(45) Veröffentlichungstag und Bekanntmachung des Hinweises auf die Patenterteilung: **14.02.2007**
(21) Anmeldenummer: 02740551.3
(22) Anmeldetag: 02.05.2002
(51) Int. Cl.: C07D 471/04, C07C 233/06

(54) **VERFAHREN ZUR HERSTELLUNG VON ZOLPIDEM**
METHOD FOR THE PRODUCTION OF ZOLPIDEM
PROCEDE DE PRODUCTION DE ZOLPIDEM

(30) Priorität: 03.05.2001 DE 10121638
(43) Veröffentlichungstag der Anmeldung: 10.03.2004
(73) Patentinhaber: Boehringer Ingelheim Pharma GmbH & Co.KG, 55216 Ingelheim am Rhein (DE)
(72) Erfinder: SAUTER, Markus, 55457 GENSINGEN (DE); WOHLLEBEN, Wolfgang, 55545 Bad Kreuznach (DE)
(86) Internationale Anmeldenummer: PCT/EP2002/004796
(87) Internationale Veröffentlichungsnummer: WO 2002/090356

(56) Entgegenhaltungen:
- EP-A- 0 251 859
- TRAPANI G ET AL: "Synthesis and Binding Affinity of 2-Phenylimidazo[1,2-alpha]pyridine Derivatives for both Central and Peripheral Benzodiazepine Receptors. A New Series of High-Affinity and Selective Ligands for the Peripheral Type" JOURNAL OF MEDICINAL CHEMISTRY, AMERICAN CHEMICAL SOCIETY. WASHINGTON, US, Bd. 40, 1997, Seiten 3109-3118, XP002900938 ISSN: 0022-2623
- TRAPANI G: "Novel 2-Phenylimidazo[1,2-a]pyridine Derivatives as Potent and Selective Ligands for Peripheral Benzodiazepine Receptors: Synthesis, Binding Affinity, and in Vivo Studies" JOURNAL OF MEDICINAL CHEMISTRY, AMERICAN CHEMICAL SOCIETY. WASHINGTON, US, Bd. 42, 1999, Seiten 3934-3941, XP002154069 ISSN: 0022-2623 in der Anmeldung erwähnt

## Beschreibung

Die Erfindung betrifft ein großtechnisch anwendbares Verfahren zur Herstellung von Zolpidem.

### Hintergrund der Erfindung

Zolpidem ist ein bekanntes Sedativum, das folgende Struktur aufweist:

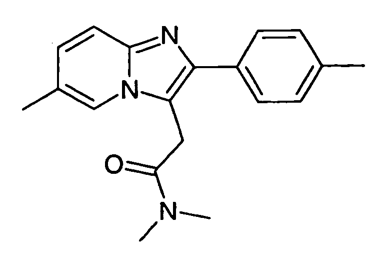

EP 0 251 859 beschreibt ein Verfahren zur Herstellung von Zolpidem. Die von einem Bromacetophenon ausgehende sechsstufige Synthese stellt ein in der Regel aufwendiges Syntheseverfahren dar.
In der Literatur (J. of Med. Chem.,1999, Vol. 42, No. 19,3934-3941) wird ein Verfahren zur Herstellung von zolpidemanalogen Verbindungen beschrieben, welches 2-Aminopyridine und entsprechende Bromoketoamide umsetzt. Ein Verfahren zur Herstellung Zolpidem-ähnlicher Verbindungen ist auch in J. of Med. Chem.,1997, Vol. 40, No. 19, 3116, beschrieben.

Es ist daher die Aufgabe der vorliegenden Erfindung ein verbessertes, großtechnisch anwendbares, kostengünstiges Verfahren zur Herstellung von Zolpidem bereitzustellen.

### Detaillierte Beschreibung der Erfindung

Die vorliegende Erfindung löst die vorstehend genannte Aufgabe über das im folgenden beschriebene Syntheseverfahren.

Gegenstand der Erfindung ist somit ein Verfahren zur Herstellung einer Verbindung der Formel (I) 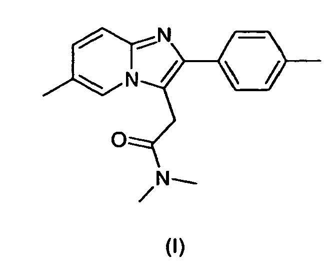 wobei,
eine Verbindung der Formel (II), 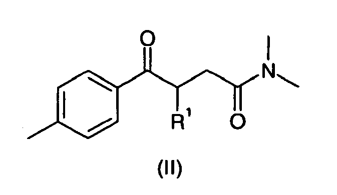 worin
R¹ für Chlor, Brom, Iod, -O-COCH₃ , -Tosylat oder -Mesylat steht,
gegebenenfalls in einem geeigneten Verdünnungsmittel und/oder in Gegenwart eines geeigneten Zusatzreagenz oder Katalysators,
mit einer Verbindung der Formel (III), 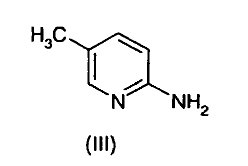 umgesetzt wird,
dadurch gekennzeichnet, dass die Reaktion in einem Temperaturbereich von 20 bis 80 °C durchgeführt wird.

Besonders bevorzugt ist ein Verfahren, worin ein Verdünnungsmittel eingesetzt wird, welches ausgewählt wird aus der Gruppe bestehend aus Acetonitril, N-Methylpyrrolidinon, Tetrahydrofuran, Aceton, Ethanol und Dichlormethan.
Weiterhin bevorzugt ist ein Verfahren, worin als Verdünnungsmittel Acetonitril eingesetzt wird.

Erfindungsgemäß von besonderer Bedeutung ist ein Verfahren, worin die Reaktion bei einer Temperatur von 60 bis 75 °C, vorzugsweise 70°C durchgeführt wird.

Ferner bevorzugt ist ein Verfahren, worin die Verbindung der Formel (II) zu der Verbindung der Formel (III) in einem molaren Verhältnis von 1:1 bis 1:3 eingesetzt wird.

Besonders bevorzugt ist ein Verfahren, worin die Verbindung der Formel (II) zu der Verbindung der Formel (III) in einem molaren Verhältnis von 1:2 eingesetzt wird.

Insbesondere bevorzugt ist ein Verfahren, worin ein Zusatzreagenz und/oder Katalysator eingesetzt wird, ausgewählt aus der Gruppe bestehend aus p-Toluolsulfonsäure Monohydrat, Natriumhydrogencarbonat, Natriumacetat, Pyridin, Dimethylaminopyridin, Magnesiumsulfat, Triethylamin, Trimethylorthoformiat und Tetrabutylammoniumbromid.

Ein weiterer Gegenstand der Erfindung ist die Verbindung der Formel (II) 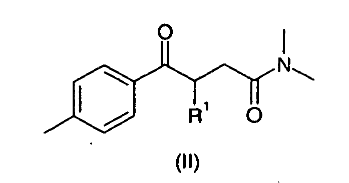 worin
R¹ für Brom steht.

Ebenfalls Gegenstand der vorliegenden Erfindung ist ein Verfahren zur Herstellung einer Verbindung der Formel (II), worin eine Verbindung der Formel (IV) 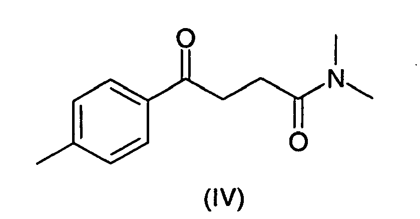 in einem Verdünnungsmittel, vorzugsweise Dichlormethan, mit elementarem Brom umgesetzt wird.
Die Umsetzung einer Verbindung der Formel (IV) mit elementarem Brom erfolgt in der Regel bei einer Temperatur von 10 bis 50 °C, vorzugsweise von 15 bis 35 °C, besonders bevorzugt von 18 bis 30 °C, insbesondere bevorzugt von etwa 20 oder 25 °C.
Dabei wird die Verbindung der Formel IV zu elementarem Brom in der Regel in einem molaren Verhältnis von 1,5 :1 bis 1:1.5, bevorzugt von etwa 1:1.2 eingesetzt.

Ferner ist ein Gegenstand der vorliegenden Erfindung die Verwendung von Verbindungen der Formel (II) zur Herstellung pharmazeutisch wirksamer Verbindungen.

Bevorzugt ist die Verwendung der Verbindung der Formel (II) zur Herstellung von Zolpidem.

Ein weiterer Gegenstand der vorliegenden Erfindung ist ein Verfahren zur Herstellung einer Verbindung der Formel (I), wobei das Verfahren folgende Schritte umfaßt:
a) Umsetzung der Verbindung der Formel (IV) in einem organischen Verdünnungsmittel bei einer Temperatur von 30 bis 50 °C, vorzugsweise etwa 40 °C, mit elementarem Brom.
b) Waschen des Reaktionsgemisches mit Wasser,
c) nach einer Phasentrennung Einengen der organischen Phase und gegebenenfalls Verdünnung mit einem weiteren organischen Verdünnungsmittel , und
d) Umsetzen der eingeengten organischen Phase ohne Isolierung des Zwischenproduktes aus a) bis c) mit der Verbindung der Formel (III) bei 20 bis 80 °C, vorzugsweise 60 bis 75 °C, bevorzugt etwa 70 °C.

Zur Salzbildung der erfindungsgemäßen Verbindungen geeignete Säuren sind beispielsweise Salzsäure, Bromwasserstoffsäure, Schwefelsäure, Phosphorsäure, Salpetersäure, Oxalsäure, Malonsäure, Fumarsäure, Maleinsäure, Weinsäure, Zitronensäure, Ascorbinsäure und Methansulfonsäure, insbesondere Weinsäure.

In einer bevorzugten Ausführungsform des erfindungsgemäßen Verfahrens zur Herstellung der Verbindung der Formel II wird ein Äquivalent der Verbindung der Formel IV in einem Verdünnungsmittel, vorzugsweise Eisessig, Ethylacetat, n-Butylacetat oder Diethylether, besonders bevorzugt Ethylacetat, gelöst. Eine Lösung aus in der Regel 1 bis 1,5 Äquivalenten, vorzugsweise einem Äquivalent, Brom wird in einem Verdünnungsmittel, vorzugsweise Ethylacetat, bei einer Temperatur von 40 bis 70 °C, vorzugsweise etwa 45 °C, zugetropft und 2 bis 24 h, vorzugsweise 5 bis 15 h, besonders bevorzugt 12 h, bei einer Temperatur von 10 bis 50 °C,
vorzugsweise von 15 bis 35 °C, besonders bevorzugt von 18 bis 30 °C, insbesondere bevorzugt von etwa 20 oder 25 °C gerührt. Die erhaltene Suspension wird filtriert , der Rückstand in wenig Wasser eingetragen und etwa 0,5 bis 2 h, vorzugsweise etwa 1 h, gerührt. Die Suspension wird erneut filtriert und der Rückstand mit Wasser gewaschen. Die erhaltenen Kristalle werden getrocknet, vorzugsweise im Vakuumtrockenschrank bei 40 bis 80 °C, bevorzugt bei etwa 70 °C.

In einer bevorzugten Ausführungsform des erfindungsgemäßen Verfahrens zur Herstellung der Verbindung der Formel I wird etwa 1 Äquivalent der Verbindung der Formel (II) in einem Verdünnungsmittel, beispielsweise Acetonitril, vorgelegt und bei 20 bis 80°C, besonders bevorzugt bei 40 bis 75 °C, insbesondere bevorzugt bei einer Temperatur von etwa 70 °C innerhalb von 0,5 bis 3 h, vorzugsweise von 1 bis 2 h, besonders bevorzugt von etwa 1,5 oder 1,75 h, eine Lösung aus in der Regel 2 Äquivalenten der Verbindung der allgemeinen Formel (III) und einem Verdünnungsmittel, beispielsweise Acetonitril, zugetropft. Nach beendeter Zugabe wird 2 bis 6 h , vorzugsweise 2 bis 5 h, besonders bevorzugt etwa 2,5 bis 3 h gerührt. Das Reaktionsgemisch wird anschließend mit einem Verdünnungsmittel,
vorzugsweise Dichlormethan verdünnt und ein- bis fünf mal, vorzugsweise dreimal mit Wasser gewaschen. Die organische Phase wird ein- bis fünfmal mit Salzsäure, vorzugsweise 2 N Salzsäure extrahiert. Die vereinigten sauren Phasen werden mittels einer Base, vorzugsweise Natronlauge, besonders bevorzugt 20%iger Natronlauge, auf einen pH-Wert zwischen etwa 7 und 9, vorzugsweise einen pH-Wert von etwa 8 eingestellt. Nach Abkühlen des Reaktionsgemisches wird ein- bis fünfmal mit einem organischen Verdünnungsmittel , ausgewählt aus der Gruppe bestehend aus Dichlormethan, Toluol, Ethylacetat, n-Butylacetat und Methyl-tert.-Butylether, vorzugsweise Dichlormethan und Ethylacetat, besonders bevorzugt Ethylacetat extrahiert. Die vereinigten organischen Phasen werden getrocknet, vorzugsweise mit Magnesiumsulfat, und eingeengt. Das kristallisierte Produkt wird mit wenig Wasser versetzt und 5 bis 20 h, vorzugsweise 15 h gerührt, die Kristalle abfiltriert, mit Wasser gewaschen und getrocknet, bevorzugt bei 30 bis 80 °C, vorzugsweise bei 60 °C, über 1 bis 10 h, vorzugsweise 5 h.

In einer bevorzugten Ausführungsform des erfindungsgemäßen Verfahrens zur Herstellung des Semitartratsalzes der Verbindung der allgemeinen Formel I werden in der Regel 2 Äquivalente der Verbindung der Formel I in einem Verdünnungsmittel, vorzugsweise Methanol, Ethylacetat, Isopropanol oder Ethanol, besonders bevorzugt Methanol, vorgelegt und eine Lösung aus 1 Äquivalent (2R, 3R)-(+)-Weinsäure in einem Verdünnungsmittel, vorzugsweise Methanol, Ethanol oder Isopropanol, besonders bevorzugt Methanol, zugegeben.
Gegebenenfalls wird ein Fällingsmittel vorzugsweise tert-Butyl-methylether, ein Isopropanol/Methanol-Gemisch oder ein Methanol/Ether-Gemisch, vorzugsweise tert-Butyl-methylether zugefügt. Es wird 1 bis 24 h, vorzugsweise 12 h, bei einer Temperatur von 15 bis 30 °C, vorzugsweise bei etwa 20 oder 25 °C, gerührt. Die entstandene Suspension weitere 0,5 bis 3h, vorzugsweise etwa 1 Stunde bei einer Temperatur von 0 bis 20 °C, vorzugsweise 3 bis 10 °C, insbesonder bevorzugt bei etwa 5°C gerührt. Die erhaltenen Kristalle werden filtriert, gegebenenfalls mit einem Lösungsmittel, vorzugsweise mit tert-Butyl-methylether gewaschen, und die Kristalle getrocknet, vorzugsweise 1 bis 10 h, besonder bevorzugt 5 Stunden bei einer Temperatur von 20 bis 70 °C, vorzugsweise von etwa 50°C.

In einer besonders bevorzugten Ausführungsform des erfindungsgemäßen Verfahrens wird etwa 1 Äquivalent der Verbindung der Formel (IV) in einem Verdünnungsmittel, beispielsweise Ethylacetat, Butylacetat oder Dichlormethan, vorzugsweise Dichlormethan, vorgelegt und auf 30 bis 50 °C, vorzugsweise 40 °C, erhitzt. Vorzugsweise werden dem Reaktionsgemisch katalytische Mengen,
bevorzugt 5 bis 6 Mol-% HBr zugefügt. Anschließend werden 1.2 Äquivalente Brom zugetropft. Das Reaktonsgemisch wird noch weitere 60 min, vorzugsweise 30 min gerührt. Der Ansatz wird auf etwa 20 bis 25 °C abgekühlt und mit Wasser extrahiert. Die organische Phase wird auf etwa 10% (v/v) eingeengt und anschließend mit einem weiteren Verdünnungsmittel, vorzugsweise Tetrahydrofuran, N-Methylpyrrolidinon oder Acetonitril, vorzugsweise Acetonitril verdünnt. Das Gemisch wird zu einer Lösung aus 1.3 Äquivalenten 6-Amino-3-picolin und einem Verdünnungsmittel, vorzugsweise Tetrahydrofuran, N-Methylpyroolidinon oder Acetonitril, vorzugsweise Acetonitril, zugetropft. Anschließend wir etwa 2 h bei 50 bis 80 °C, vorzugsweise 70 °C, gerührt. Das Reaktionsgemisch wird mit einem organischen Verdünnungsmittel, vorzugsweise Toluol, versetzt, mit einer wässrigen Lösung, beispielsweise 2N Salzsäure, extrahiert und die organische Phase verworfen. Die wäßrige Phase wird erneut mit organischem Verdünnungsmittel versetzt, auf einen pH-Wert von etwa 4 eingestellt, und die organische Phase erneut verworfen. Der Extraktionsschritt wird bei einem pH-Wert von etwa 8 bis 9 wiederholt. Nach Abtrennung der wäßrigen Phase wird die organische Phase auf etwa 10% eingeengt. Der Rückstand wird mit Diisopropylether, Diethylether oder Methyl-tert.Butylether, vorzugsweise Methyl-tert.Butylether, versetzt und etwa 30 bis 60 Minuten bei etwa 0 bis 15°C, vorzugsweise 5 °C, gerührt. Die entstandenen Kristalle werden gewaschen und getrocknet.

Die erfindüngsgemäße Vorgehensweise führt zu einem kostengünstigen Verfahren mit einer hohen Raum-Zeit-Ausbeute bezüglich der Verbindung der Formel I bzw. deren pharmakologisch verträglichen Salze sowie einer hohen Ausbeute und Reinheit des Zwischenprodukts der Formel II, welches ohne Isolierung oder chromatographische Aufreinigung weiterverärbeitet werden kann.

Die nachfolgenden Beispiele dienen der Illustration exemplarisch durchgeführter Verfahren zur Herstellung der Verbindung der Formel I. Sie sind lediglich als mögliche, exemplarisch dargestellte Vorgehensweisen zu verstehen, ohne die Erfindung auf deren Inhalt zu beschränken.

### Beispiel 1

### 3-(4-Methyl-benzoyl)-2-brom-propyl-dimethylamid

18,6 g (84,8 mmol) 3-(4-Methyl-benzoyl)-propyl-dimethylamid werden in 50 ml Eisessig gelöst. Bei Raumtemperatur wird innerhalb von 50 Minuten eine Lösung aus 13,55 g (84,8 mmol) Brom und 45 ml Eisessig zugetropft und anschließend über Nacht gerührt.
Die entstandene Suspension wird filtriert und mit 30 ml Eisessig gewaschen. Der Filterrückstand wird in 200 ml destilliertes Wasser eingetragen, gut verrieben und 1 Stunde gerührt. Das Produkt wird erneut filtriert und mit weiteren 200 ml Wasser gewaschen. Die erhaltenen Kristalle (21,16 g) werden 6 Stunden im Vakuumtrockenschrank bei 70°C getrocknet.
Ausbeute 18,18 g weiße Kristalle (71,9 % d. Th.)
Schmelzpunkt: 119 - 121 °C

### Beispiel 2

### N,N-6-Trimethyl-2-(4-methylphenyl)imidazo[1,2-a]pyridine-3-acetamid

50 g (167,7 mmol) 3-(4-Methyl-benzoyl)-2-brom-propyl-dimethylamid werden in 500 ml Acetonitil vorgelegt. Bei 60°C wird innerhalb von 1,75 Stunden eine Lösung aus 36,27 g (335,4 mmol) 6-Amino-3-picolin und 350 ml Acetonitril zugetropft und nach beendeter Zugabe weitere 4 Stunden gerührt. Die erhaltene Lösung wird mit 1000 ml Dichlormethan verdünnt und dreimal mit je 2000 ml destilliertem Wasser gewaschen. Anschließend wird die organische Phase dreimal mit je 1000 ml 2N Salzsäure extrahiert. Die vereinigten sauren Phasen werden mit 20 %iger Natronlauge auf pH 8 eingestellt und nach vorherigem Abkühlen dreimal mit je 11 Dichlormethan extrahiert. Diese organischen Phasen werden vereinigt, mit Magnesiumsulfat getrocknet und eingeengt. Die erhaltenen Kristalle werden mit 500 ml destilliertem Wasser verrieben, über Nacht gerührt, abfiltriert, mit 50 ml destilliertem Wasser nachgewaschen und der Rückstand 5 Stunden bei 60°C im Vakuumtrockenschrank getrocknet.
Ausbeute: 17,94 g hellbraune Kristalle (45,7 % d. Th).

### Beispiel 3

### N, N-6-Trimethyl-2-(4-methylphenyl)imidazo[1,2-a]pyridine-3-acetamid

10,0 g (33,5 mmol) 3-(4-Methyl-benzoyl)-2-Brom-propyl-dimethylamid und 7,25 g (67,0 mmol) 6-Amino-3-picolin werden in 170 ml 1,3-Dimethyl-2-imidazolidinone gelöst und 3 Stunden bei 60°C gerührt. Das Reaktionsgemisch wird abgekühlt und mit 100 ml Dichlormethan verdünnt. Anschließend wird fünfmal mit je 150 ml destilliertem Wasser gewaschen. Die organische Phase wird zweimal mit je 150 ml 2N Salzsäure gewaschen. Die vereinigten sauren Phasen werden mit 2N Natronlauge auf pH 8 eingestellt. Es wird zweimal mit je 150 ml Dichlormethan extrahiert, die organischen Phasen mit MgSO₄ getrocknet und eingeengt.
Das erhaltene braune Öl wird mit 50 ml n-Heptan versetzt und 30 Minuten gerührt. Das überstehende Verdünnungsmittel wird vom ausfallenden Produkt abdekantiert und dieses zweimal mit je 10 ml n-Heptan nachgewaschen.Der Rückstand wird nochmals eingeengt, mit 200 ml destilliertem Wasser versetzt und 30 Minuten gerührt. Das Produkt wird abfiltriert, mit 50 ml destilliertem Wasser gewaschen und getrocknet.
Ausbeute: 2,38 g beige Kristalle (23,1 % d. Th.)
Schmelzpunkt: 194 - 195°C

### Beispiel 4

### N,N-6-Trimethyl-2-(4-methylphenyl)imidazo[1,2-a]pyridine-3-acetamid Semitartrat

17,94 g (94 %ig) (54,9 mmol) N,N-6-Trimethyl-2-(4-methylphenyl)imidazo[1,2-a]pyridine-3-acetamid werden in 90 ml Methanol vorgelegt. Es wird eine Lösung aus 4,13 g (27,5 mmol) (2R, 3R)-(+)-Weinsäure und 125 ml Methanol und anschließend 28 ml Methyl-tert-butyl-ether (MTBE) innerhalb von 30 Sekunden zugegeben. Es wird 15 h bei Raumtemperatur gerührt. Die entstandene hellbraune Suspension wird noch 1 Stunde bei 5°C gerührt, abfiltriert, der Rückstand mit 50 ml MTBE gewaschen, und die Kristalle 5 Stunden im Vakuumtrockenschrank bei 50°C getrocknet.
Ausbeute: 18,3 g Kristalle (87,2 % d. Th.)

### Beispiel 5

### N,N-6-Trimethyl-2-(4-methylphenyl)imidazo[1,2-a]pyridine-3-acetamid

100 g (0.456 mol) 3-(4-Methyl-benzoyl)-propyl-dimethylamid werden in 400 ml Dichlormethan gelöst. In die Lösung wird 2 g (0.025 mol) Bromwasserstoff eingeleitet und zum Rückfluß erhitzt. Anschließend wird innerhalb von 45 Minuten 86,1 g (0,539 mol) Brom zugetropft und 30 min gerührt. Der Ansatz wird auf Raumtemperatur abgekühlt und mit 600 ml destilliertem Wasser gewaschen. Die wässrige Phase wird verworfen. Die organische Phase wird auf etwa 10 % (v/v) eingeengt und anschließend mit 300 ml Acetonitril verdünnt. Diese Lösung wird innerhalb von 45 min zu einer Lösung aus 66,62 g (0,616 mol) 6-Amino-3-picolin in 150 ml Acetonitril bei 70 °C zugetropft und 1,5 h gerührt. Danach werden bei 20-30 °C 400 ml Toluol zugegeben und anschließend mit 500 ml 2N Salzsäure extrahiert. Die Toluolphase wird verworfen, die wäßrige Phase erneut mit 400 ml Toluol versetzt und mit 20%iger Natronlauge auf pH 4 eingestellt. Die Toluolphase wird verworfen, die wäßrige Phase mit 400 ml Toluol versetzt und mit 20%iger Natronlauge auf pH 8,5 eingestellt. Die Toluolphase wird abgetrennt und auf 10 % (v/v) eingeengt. Der Rückstand wird mit MTBE versetzt und 2 h bei 5°C gerührt. Die entstandenen Kristalle werden abgesaugt, mit MTBE gewaschen und getrocknet. Ausbeute: 43 g Zolpidem (30,7 %).

## Patentansprüche

1. Verfahren zur Herstellung einer Verbindung der Formel (I) 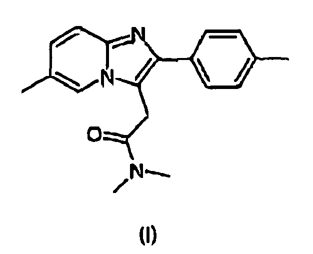 wobei,
eine Verbindung der Formel (II), 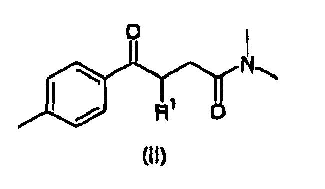 worin
R¹ für Chlor, Brom, Iod, -O-COCH₃, -Tosylat oder -Mesylat steht,
gegebenenfalls in einem geeigneten Verdünnungsmittel und/oder in Gegenwart eines geeigneten Zusatzreagenz oder Katalysators,
mit einer Verbindung der Formel (III), 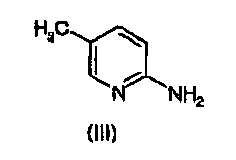 umgesetzt wird,
**dadurch gekennzeichnet, dass** die Reaktion in einem Temperaturbereich von 20 bis 80 °C durchgeführt wird.

2. Verfahren nach Anspruch 1, **dadurch geKennzeichnet, dass** ein Verdünnungsmittel eingesetzt wird, welches ausgewählt wird aus der Gruppe bestehend aus Acetonitril, N-Methylpyrrolidinon, Tetrahydrofuran, Aceton, Ethanol und Dichlormethan.

3. Verfahren nach einem der Ansprüche 1 bis 2, **dadurch gekennzeichnet, dass** als Verdünnungsmittel Acetonitril eingesetzt wird.

4. Verfahren nach einem der Ansprüche 1 bis 3, **dadurch gekennzeichnet, dass** die Reaktion bei einer Temperatur von 60 bis 75 °C durchgeführt wird.

5. Verfahren nach einem der Ansprüche 1 bis 4, **dadurch gekennzeichnet, dass** die Verbindung der Formel (II) zu der Verbindung der Formel (III) in einem molaren Verhältnis von 1:1 bis 1:3 eingesetzt wird.

6. Verfahren nach einem der Ansprüche 1 bis 5, **dadurch gekennzeichnet, dass** die Verbindung der Formel (II) zu der Verbindung der Formel (III) in einem molaren Verhältnis von 1:2 eingesetzt wird.

7. Verfahren nach einem der Ansprüche 1 bis 6, **dadurch gekennzeichnet, dass** ein Zusatzreagenz und/oder Katalysator eingesetzt wird, ausgewählt aus der Gruppe bestehend aus p-Toluolsulfonsäure Monohydrat, Natriumhydrogencarbonat, Natriumacetat, Pyridin, Dimethylaminopyridin, Magnesiumsulfat, Triethylamin, Trimethylorthoformiat und Tetrabutylammoniumbromid.

8. Verbindung der Formel (II) 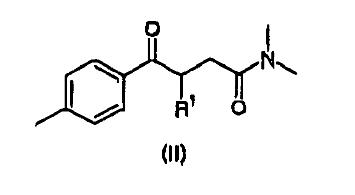 worin
R¹ für Brom steht,

9. Verfahren zur Herstellung einer Verbindung der Formel (II) gemäß Anspruch 8,
**dadurch gekennzeichnet, dass**
eine Verbindung der Formel (IV) 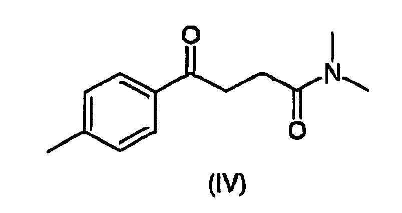 in einem Verdünnungsmittel mit elementarem Brom umgesetzt wird.

10. Verfahren nach Anspruch 9, wobei das Verdünnungsmittel Dichlormethan ist.

11. Verwendung der Verbindung der Formel (II) gemäß Anspruch 8 zur Herstellung pharmazeutisch wirksamer Verbindungen.

12. Verwendung nach Anspruch 11 der Verbindung der Formel (II) zur Herstellung von Zolpidem.

13. Verfahren zur Herstellung einer Verbindung der Formel (I), wobei das Verfahren folgende Schritte umfaßt:
a) Umsetzung der Verbindung der Formel (IV) in einem organischen Verdünnungsmittel bei einer Temperatur von 30 bis 50 °C mit elementarem Brom,
b) Waschen des Reaktionsgemisches mit Wasser,
c) nach einer Phasentrennung Einengen der organischen Phase und gegebenenfalls Verdünnung mit einem weiteren organischen Verdünnungsmittel, und
d) Umsetzen der eingeengten organischen Phase ohne Isolierung des Zwischenproduktes aus a) bis c) mit der Verbindung der Formel (III) bei 20 bis 80 °C.

## Claims

1. Process for preparing a compound of formula (I) 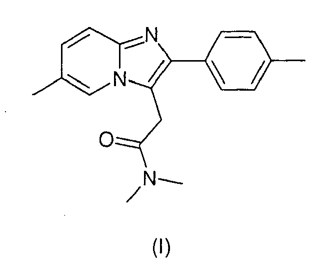 wherein
a compound of formula (II) 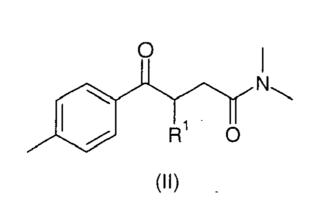 wherein
R¹ denotes chlorine, bromine, iodine, -O-COCH₃, tosylate or mesylate,
is reacted with a compound of formula (III), 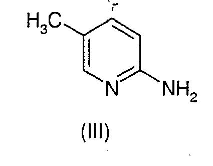 optionally in a suitable diluent and/or in the presence of a suitable added reagent or catalyst,
**characterised in that** the reaction is carried out in a temperature range from 20 to 80°C.

2. Process according to claim 1, **characterised in that** a diluent is used which is selected from among acetonitrile, N-methylpyrrolidinone, tetrahydrofuran, acetone, ethanol and dichloromethane.

3. Process according to one of claims 1 to 2, **characterised in that** acetonitrile is used as diluent.

4. Process according to one of claims 1 to 3, **characterised in that** the reaction is carried out at a temperature of 60 to 75°C.

5. Process according to one of claims 1 to 4, **characterised in that** the compound of formula (II) is used in a molar ratio of 1:1 to 1:3 to the compound of formula (III).

6. Process according to one of claims 1 to 5, **characterised in that** the compound of formula (II) is used in a molar ratio of about 1:2 to the compound of formula (III).

7. Process according to one of claims 1 to 6, **characterised in that** an added reagent and/or catalyst is used, selected from among p-toluenesulphonic acid monohydrate, sodium hydrogen carbonate, sodium acetate, pyridine, dimethylaminopyridine, magnesium sulphate, triethylamine, trimethylorthoformate and tetrabutylammonium bromide.

8. Compound of formula (II) 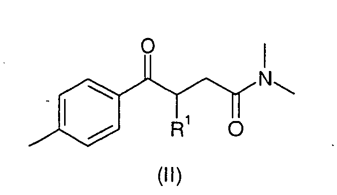 wherein
R¹ denotes bromine.

9. Process for preparing a compound of formula (II) according to claim 8,
**characterised in that**
a compound of formula (IV) 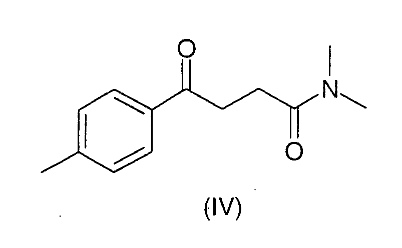 is reacted with elemental bromine in a diluent.

10. Process according to claim 9, wherein the diluent is dichloromethane.

11. Use of the compound of formula (II) according to claim 8 for preparing pharmaceutically active compounds.

12. Use according to claim 11 of the compound of formula (II) for preparing zolpidem.

13. Process for preparing a compound of formula (I), the process comprising the following steps:
a) reacting the compound of formula (IV) with elemental bromine in an organic diluent at a temperature of 30 to 50°C,
b) washing the reaction mixture with water,
c) after phase separation, concentrating the organic phase by evaporation and optionally diluting it with another organic diluent, and
d) reacting the concentrated organic phase with the compound of formula (III) at 20 to 80°C without isolating the intermediate product of steps a) to c).

## Revendications

1. Procédé de production d'un composé de formule (I) 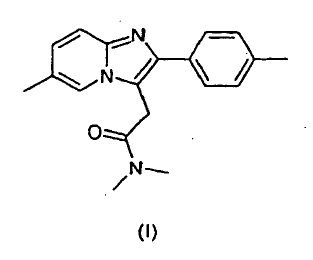 où
un composé de formule (II) 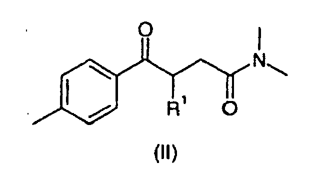 où
R¹ représente le chlore, le brome, l'iode, -O-COCH₃, tosylate ou mésylate, éventuellement dans un diluant approprié et/ou en présence d'un réactif ajouté ou catalyseur approprié,
est mis à réagir avec un composé de formule (III) 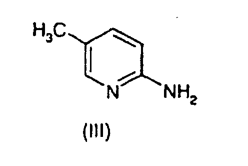 **caractérisé en ce que** la réaction est conduite dans un domaine de température de 20 à 80°C.

2. Procédé selon la revendication 1 **caractérisé en ce que** l'on utilise un diluant qui est choisi dans le groupe consistant en l'acétonitrile, la N-méthylpyrrolidinone, le tétrahydrofurane, l'acétone, l'éthanol et le dichlorométhane.

3. Procédé selon l'une des revendications 1 à 2 **caractérisé en ce que** l'acétonitrile est utilisé comme diluant.

4. Procédé selon l'une des revendications 1 à 3 **caractérisé en ce que** la réaction est conduite à une température de 60 à 75°C.

5. Procédé selon l'une des revendications 1 à 4 **caractérisé en ce que** le composé de formule (II) est utilisé par rapport au composé de formule (III) dans un rapport molaire de 1 : 1 à 1 : 3.

6. Procédé selon l'une des revendications 1 à 5 **caractérisé en ce que** le composé de formule (II) est utilisé par rapport au composé de formule (III) dans un rapport molaire de 1 : 2.

7. Procédé selon l'une des revendications 1 à 6 **caractérisé en ce que** l'on utilise un réactif ajouté et/ou catalyseur choisi dans le groupe consistant en l'acide p-toluènesulfonique monohydraté, l'hydrogénocarbonate de sodium, l'acétate de sodium, la pyridine, la diméthylaminopyridine, le sulfate de magnésium, la triéthylamine, l'orthoformiate de triméthyle et le bromure de tétrabutylammonium.

8. Composé de formule (II) 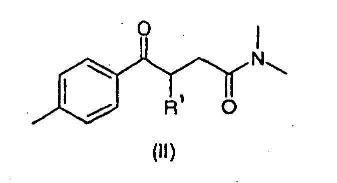 où
R¹ représente le brome.

9. Procédé de production d'un composé de formule (II) selon la revendication 8 **caractérisé en ce que**
un composé de formule (IV) 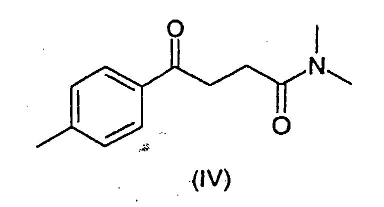 est mis à réagir avec du brome élémentaire dans un diluant.

10. Procédé selon la revendication 9 où le diluant est le dichlorométhane.

11. Utilisation du composé de formule (II) selon la revendication 8 pour la production de composés pharmaceutiquement actifs.

12. Utilisation selon la revendication 11 du composé de formule (II) pour la production du zolpidem.

13. Procédé de production d'un composé de formule (I) où le procédé comprend les étapes suivantes :
a) réaction du composé de formule (IV) dans un diluant organique à une température de 30 à 50°C avec du brome élémentaire,
b) lavage du mélange réactionnel avec de l'eau,
c) après une séparation des phases, concentration de la phase organique et éventuellement dilution avec un autre diluant organique, et
d) réaction de la phase organique concentrée sans isolement du produit intermédiaire de a) à c) avec le composé de formule (III) à 20 à 80°C.
